# FASCICULE DE BREVET EUROPEEN

(11) **EP 0 216 743 B1**
(45) Date de publication et mention de la délivrance du brevet: **17.03.1993**
(21) Numéro de dépôt: 86870129.3
(22) Date de dépôt: 17.09.1986
(51) Int. Cl.: A61K 31/275, A61K 9/52

(54) **Nouvelles formes galéniques du vérapamil, leur fabrication et médicaments contenant ces nouvelles formes galéniques**
Galenische Formen des Verapamils, deren Herstellung sowie diese enthaltende Arzneimittel
Galenical forms of verapamil, preparation thereof and medicaments containing them

(30) Priorité: 18.09.1985 LU 86077
(43) Date de publication de la demande: 01.04.1987
(73) Titulaire: "PHARLYSE", Société Anonyme, Luxembourg (LU)
(72) Inventeur: de Boeck, Arthur, B-1540 Herne (BE); Baudier, Philippe, B-1410 Waterloo (BE); Fossion, Jacques, B-1428 Braine-L'Alleud (BE)
(74) Mandataire: Fobe, Edouard (BE)

(56) Documents cités:
- EP-A- 0 063 266
- EP-A- 0 080 341
- EP-A- 0 142 877
- EP-A- 0 153 104
- WO-A- 8/30009
- GB-A- 2 056 278
- CHEMICAL ABSTRACTS, vol. 102, no. 24, 17 juin 1985, page 349, résumé no. 209280k, Columbus, Ohio, US; A. KANNIKOSKI et al.: "Release of verapamil hydrochloride from granules coated with ethyl cellulose films: part I"
- CHEMICAL ABSTRACTS, vol. 102, no. 24, 17 juin 1985, page 348, résumé no. 209279s, Columbus, Ohio, US; A. KANNIKOSKI: "Release of verapamil hydrochloride from sustained-release tablets coated with ethyl cellulose films modified with hydroxypropyl methyl cellulose"

## Description

La présente invention concerne de nouvelles formes galéniques du vérapamil, leur fabrication et les médicaments contenant les nouvelles formes galéniques.

Le vérapamil ou 5-(3,4-diméthoxyphényléthyl) méthylamino-2-(3,4-diméthoxyphényl)-2-isopropyl valéronitrile de formule I est connu depuis plus de 20 ans et sa synthèse est décrite dans le brevet belge n° 615.816 correspondant au brevet U.S. Dengel n° 3.261.859. 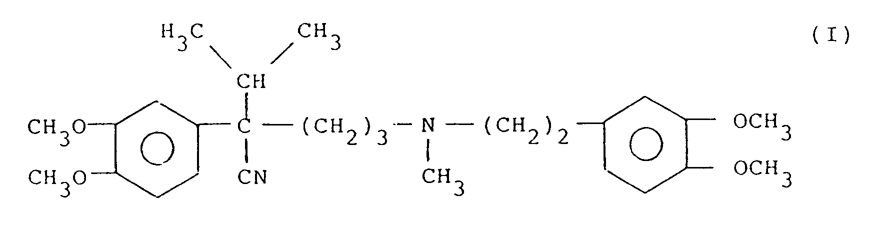
Le chlorhydrate de vérapamil est utilisé en médecine pour ses remarquables propriétés d'antagoniste de la pénétration intracellulaire du calcium. Ceci en fait un médicament intéressant dans l'angine de poitrine lorsque la crise est liée à un spasme coronarien et lorsque des effets indésirables des produits beta-adrénolytiques, tels que le propranolol, le timolol, l'aténolol et le pindolol, sont à craindre. Il est également utile dans le traitement de l'hypertension et des troubles du rythme cardiaque.

Il est connu de l'homme de l'art que l'action pharmacologique du vérapamil est proportionnelle à sa concentration plasmatique (Br J. Clin. Pharmac (1981), 12, 397 - 400) et que la zone thérapeutique optimale est comprise entre 100 ng/ml et 400 ng/ml de plasma.

L'inconvénient majeur rencontré lors d'un traitement à base de vérapamil est dû à son temps de demi-vie plasmatique très court (2 à 4 heures), ce qui nécessite plusieurs administrations quotidiennes, espacées de 6 heures seulement. Des temps d'administration aussi rapprochés rendent le traitement fort astreignant, voire impossible à suivre, surtout la nuit. De plus, on constate, après chaque administration d'une forme galénique de vérapamil à libération immédiate, c'est-à-dire en général quatre fois par jour, une succession de croissances et de décroissances rapides des taux plasmatiques. L'organisme et notamment l'organe-cible, en l'occurrence le coeur, sont ainsi soumis alternativement à des surdosages et à des sous-dosages du médicament.

Afin de pallier ces inconvénients, une première forme galénique à libération prolongée de vérapamil, dénommée "Isoptine Retard ® " se présentant sous la forme d'un comprimé constitué d'une matrice hydrophile, a été mise sur le marché. Bien que cette forme galénique à libération prolongée permette d'éliminer les pics de concentrations, la biodisponibilité du vérapamil au départ de cette forme galénique est extrêmement faible, comme on le verra plus loin, de telle sorte qu'elle ne permet pratiquement pas d'atteindre les taux plasmatiques thérapeutiques et rend par conséquent difficile l'obtention de l'effet clinique satisfaisant.

On connaît, par le document EP-A-0 080 341, une composition pharmaceutique à libération contrôlée de substances telles que le vérapamil, contenant des particules constituées de grains homogènes, ces grains étant munis d'un revêtement qui est sensiblement résistant aux conditions gastriques mais qui est érodable dans l'intestin.

Le revêtement des grains peut contenir des polymères acryliques tels que les Eudragit® S12,5, S12,5P, L12,5 ou L30D (polymérisat anionique d'acide méthacrylique et d'ester méthylique d'acide méthacrylique). Ces polymères sont des polymères entérosolubles puisque le revêtement doit être érodable dans l'intestin.

Ce document EP-A-0 080 341 décrit en fait une forme galénique libérant au moins 90 % de la substance active en une heure lorsque le pH du milieu est supérieur à 7,5 , c'est-à-dire une forme galénique à libération retardée et non une forme galénique à libération prolongée.

On connaît également, par le document EP-A-0 153 104, des formes galéniques constituées d'unités individuelles contenant une substance active, telle que du vérapamil, ces unités étant revêtues d'un film. Ce document ne décrit pas l'importance de l'utilisation d'au moins un mouillant avec un sel d'addition du vérapamil avec un acide.

De plus, les exemples de ce document dans lesquels des unités individuelles sont revêtues d'un film contenant de l'Eudragit® E30D (copolymérisat neutre ester éthylique d'acide acrylique / ester méthylique d'acide méthacrylique dans le rapport 70:30 et de poids moléculaire supérieur à 800.000) montrent l'existence de pic de concentration ou de variation importante de la concentration de la substance active. En fait, les formes galéniques selon ce document peuvent être considérées comme des formes galéniques à libération immédiate.

La présente invention a pour objet de nouvelles formes galéniques de vérapamil à libération prolongée de cette substance active, ces nouvelles formes galéniques présentant une biodisponibilité excellente tout en évitant les pics de concentrations plasmatiques, ce qui rend maintenant possible à la fois le maintien des concentrations plasmatiques en vérapamil dans la zone réellement efficace et la simplification de la posologie, puisque ces nouvelles formes ne nécessitent plus qu'une ou deux administrations quotidiennes du médicament en fonction du métabolisme du patient.

Conformément à la présente invention, les nouvelles formes galéniques à libération prolongée du vérapamil sont constituées de microgranules contenant un sel pharmacologiquement acceptable d'addition du vérapamil avec un acide, tel que le chlorhydrate de vérapamil, choisi parmi le saccharose, la polyvinylpyrrolidone, les esters d'acides gras en C₁₂ à C₂₀ du saccharose et du xylose, les glycérides du saccharose, les esters gras et de polyoxyéthylène, les éthers de sorbitane, les esters de sorbitane, les esters de sorbitane polyoxyéthylénés, les glycérides-polyglycides, les esters d'alcools-polyglycides et les lécithines, ces microgranules étant enrobés d'une membrane microporeuse constituée d'au moins un polymère acrylique choisi parmi les polyacrylates et polyméthacrylates du type Eudragit® E30D, associé à au moins un adjuvant choisi parmi les plastifiants, pigments, charges, agents mouillants, agents lubrifiants et agents anti-mousse.

Les microgranules contenant la substance active se présentent sous forme de sphérules, dont le diamètre est compris entre 0,05 mm et 3 mm, de préférence entre 0,1 mm et 2 mm.

Parmi les mouillants associés au chlorhydrate de vérapamil dans les microgranules, on peut citer, en particulier, les suivants :
- le saccharose, le mannitol, le sorbitol ;
- les lécithines ;
- les polyvinylpyrrolidones ;
- les esters d'acide gras en C₁₂ à C₂₀ du saccharose, commercialisés sous le nom de sucroesters (Gattefossé, France) ou sous le nom de crodesters (Croda, U.K.) ;
- les glycérides en C₁₂-C₂₀ du saccharose ou sucro-glycérides ;
- les esters de xylose ou xylites ;
- les glycérides polyoxyéthyléniques ;
- les esters d'acides gras et de polyoxyéthylène (Tefose® de Gattefossé, France - Crémophores® de BASF, RFA) ;
- les éthers d'alcool gras et de polyoxyéthylène (Brijs, Renex® et Eumulgines®, Henkel, RFA) ;
- les esters d'acides gras du sorbitane (Spans®, Atlas, U.S.A.) ;
- les esters polyoxyéthylènes d'acides gras du sorbitane (Tweens®, Atlas, U.S.A.) ;
- les glycérides - polyglycides et esters d'alcools - polyglycides (Gelucires®, Gattefossé, France).

Outre au moins un des mouillants précités, les microgranules peuvent contenir des excipients, tels que :
- les celluloses microcristallines, telles que les produits Avicel® (FMC, U.S.A.) ;
- les méthylcelluloses, éthylcelluloses (Ethocel® ou Aqua-Coat), carboxyméthylcelluloses, hydroxyéthylcelluloses (Natrosol®, Hercules, U.S.A.), hydroxypropylcelluloses (Klucels®, Hercules, U.S.A.) ;
- les amidons.

Les polymères constituant la membrane microporeuse peuvent être associés dans la membrane microporeuse à au moins un adjuvant choisi parmi les suivants :
- plastifiants, tels que la triacétine, le dibutylphtalate, le dibutylsébaçate, les esters d'acide citrique, les polyéthylèneglycols, les polypropylèneglycols et la polyvinylpyrrolidone ;
- pigments éventuellement colorés, tels que les oxydes de fer et l'oxyde de titane ;
- charges, telles que le lactose et le saccharose ;
- mouillants, tels que les agents tensio-actifs des types Span® et Tween®, à savoir des esters partiels d'acides gras (acides laurique, palmitique, stéarique et oléique) et d'anhydrides d'hexitols dérivés du sorbitol contenant éventuellement des chaînes polyoxyéthyléniques, de préférence les agents tensio-actifs du type Tween®, notamment le Tween 80®, ainsi que les polyéthylèneglycols ;
- lubrifiants, tels que le stéarate de magnésium et le talc ;
- agents anti-mousse, tels que l'huile de silicone ;
- agents antistatiques, tels que l'oxyde d'aluminium colloîdal.

Comme autres adjuvants éventuellement utilisés conjointement avec les polymères formant la membrane microporeuse, on peut faire usage d'agents désintégrants tels que les amidons et leurs dérivés.

Outre le ou les polymères, la membrane microporeuse contient, de préférence, du talc et/ou du stéarate de magnésium à titre de lubrifiant, de la polyvinylpyrrolidone à titre de plastifiant, du bioxyde de titane à titre de pigment, du Tween 80® comme mouillant, de l'oxyde d'aluminium colloîdal comme agent antistatique et de l'huile de silicone comme agent anti-mousse.

Le poids de la membrane microporeuse peut être de 2 à 35 %, de préférence de 5 à 22 % de celui des microgranules. Ces derniers peuvent contenir du chlorhydrate de vérapamil à raison de 20 à 95 % en poids, de préférence de 30 à 85 % en poids.

La membrane microporeuse peut contenir de 5 à 95 % et, de préférence, de 30 à 90 % de polymères ou mélange de polymères.

L'invention concerne également un médicament contenant du vérapamil à titre d'ingrédient à libération prolongée, ce médicament étant constitué de microgranules contenant du vérapamil, de préférence sous forme de chlorhydrate et au moins un mouillant, enrobés d'une membrane microporeuse à base d'au moins un polymère, ces microgranules enrobés étant contenus dans des gélules, sachets ou distributeurs de doses.

La présente invention concerne également un procédé d'obtention des nouvelles formes du vérapamil à libération retardée et/ou prolongée dans le tractus gastro-intestinal, ce procédé consistant à préparer au préalable des microgranules et à les enrober d'une membrane microporeuse.

Diverses techniques connues sont utilisables pour obtenir les microgranules contenant du chlorhydrate de vérapamil. Une première technique consiste à mélanger du chlorhydrate de vérapamil avec le ou les mouillants choisis sous forme finement divisée ou fondue, ou en solution, en présence d'un solvant, tel que de l'eau ou un mélange hydroalcoolique, de manière à obtenir une masse plastique ou pâte extrudable.

Cette pâte est ensuite extrudée dans une extrudeuse et le produit extrudé est rendu sphérique. On utilise de préférence une extrudeuse à filière d'un diamètre compris entre 0,1 mm et 3 mm, de préférence entre 0,5 mm et 2 mm. Divers types d'extrudeuses sont utilisables, par exemple l'extrudeuse de la firme ALEXANDERWERK (RFA) ou l'appareil dénommé X-truder® de la firme FUJI-PAUDAL (Japon). Pour obtenir des microsphères ou microgranules à partir du produit extrudé se présentant sous forme de petits cylindres, on peut utiliser un appareil dit "sphéroniseur" du type CALEVA (Grande-Bretagne) ou MARUMERIZER (de la firme FUJI-PAUDAL - Japon).

Une autre technique d'obtention des microgranules consiste à pistoler et/ou à saupoudrer des noyaux obtenus par agglomération de chlorhydrate de vérapamil mélangé éventuellement à au moins un mouillant à l'aide d'une solution ou dispersion aqueuse ou organique d'au moins un mouillant, par exemple dans une turbine connue de dragéification ou un appareil de granulation, tel que le CF granulator system de la firme FREUND INDUSTRIAL CO. (Japon) ou encore dans un granulateur connu du type planétaire.

Les microgranules obtenus sont séchés par un moyen quelconque, par exemple dans une étuve ou à l'aide d'un gaz dans un lit fluidisé.

Enfin, les microgranules sont calibrés au diamètre voulu par passage à travers des tamis appropriés.

La membrane microporeuse peut être appliquée sur les microgranules en pulvérisant une dispersion aqueuse ou hydroalcoolique d'au moins un des polymères précités et d'au moins un des adjuvants susdits sur les microgranules. Cette pulvérisation peut s'effectuer par pistolage ou par pulvérisation de la dispersion susdite dans une turbine ou dans un lit fluidisé.

Selon une forme de réalisation exemplative de l'invention, la membrane microporeuse peut être obtenue au départ d'une dispersion aqueuse contenant en poids :

| | |
|---|---|
| 10 à 45 % | d'Eudragit E30D® (polymère) ; |
| 5 à 70 % | d'Eudragit L30D® (polymère) ; |
| 1 à 25 % | de talc (lubrifiant) ; |
| 1 à 15 % | de polyvinylpyrrolidone (plastifiant) ; |
| 0,05 à 4 % | d'oxyde d'aluminium colloîdal (agent anti-mousse), ou |
| 0,01 à 2 % | d'huile de silicone (agent anti-mousse) |
| 10 à 70 % | d'eau (véhicule). |

L'ensemble des caractéristiques et avantages de l'invention sera mieux compris par l'homme de l'art en se référant à la description qui va suivre de modes de réalisation particuliers donnés à titre d'exemples non limitatifs de la nouvelle forme galénique du vérapamil, de son procédé de fabrication et de ses applications thérapeutiques, en particulier en relation avec des contrôles pharmacocinétiques utilisant cette nouvelle forme galénique.

### Exemples de préparation de la nouvelle forme galénique

### EXEMPLE 1

### a. Fabrication des microgranules

On a utilisé les ingrédients suivants :

| | |
|---|---|
| Vérapamil HCl | 325 g |
| Saccharose | 125 g |
| Avicel PH 101 ® | 25 g |
| Methocel E5 ® | 20 g |
| Polyvinylpyrrolidone K30 | 5 g |
| | 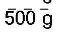 |

Après avoir introduit les poudres dans un mélangeur planétaire et les avoir granulées par addition de 75 g d'eau distillée, la masse plastique obtenue a été extrudée au travers des trous d'un diamètre de 1 mm d'une filière cylindrique d'une extrudeuse (Alexanderwerk). Les petits cylindres obtenus ont été ensuite rendus sphériques par sphéronisation dans un appareil de type Marumerizer. Après séchage pendant 24 h dans une étuve ventilée portée à 50°C, la fraction de microgranules dont le diamètre est compris entre 0,7 mm et 1,4 mm a été récupérée par tamisage au travers de tamis appropriés. On a ainsi obtenu 437 g de microgranules (rendement : 87 %).

### b. Enrobage des microgranules

Sur 400 g de microgranules, dont la granulation est comprise entre 0,7 et 1,4 mm, on a pistolé dans un appareil à lit fluidisé de marque Aéromatic type Strea I, 176 g de la dispersion suivante :

| | |
|---|---|
| stéarate de magnésium | 7,50 g |
| bioxyde de titane | 6,25 g |
| polyvinylpyrrolidone | 6,25 g |
| Tween 20 ® | 0,20 g |
| chlorhydrate de vérapamil | 6,25 g |
| eau | 145,25 g |
| Eudragit E30D ® | 328,30 g |
| | 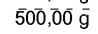 |
| Durée du pistolage : 45 minutes Séchage dans étuve à 50°C pendant 24 heures. | |

On notera que le chlorhydrate de vérapamil contenu dans la membrane appliquée sur les microgranules est destiné à être libéré rapidement après l'administration de la forme pharmaceutique.

### c. Formes pharmaceutiques

Après tamisage et contrôle du titre en chlorhydrate de vérapamil des microgranules et après avoir ajusté ce titre à la valeur souhaitée par addition de microgranules neutres, tels que des microgranules de saccharose, le mélange a été introduit dans des gélules de gélatine dure pour obtenir le dosage unitaire de vérapamil souhaité.

### d. Mesures de la vitesse de libération du vérapamil hors des microgranules

Pour vérifier la qualité de la préparation à libération prolongée du vérapamil, on a utilisé la méthode décrite dans "The United States Pharmacopoeia (USP) XXI", édition 1985, p 1243-1244 sous le titre : Dissolution Apparatus II, le milieu de dissolution étant constitué d'un tampon de pH = 5,8. On a utilisé 251,5 mg de microgranules de l'exemple 1 par ballon.

Les résultats suivants ont été obtenus dans ladite dissolution.

| Temps | % de vérapamil HCL dissous ± SD (déviation standard) |
|---|---|
| Après 1 h | 1,6 ± 0,2 |
| Après 4 h | 27,0 ± 0,3 |
| Après 8 h | 84,2 ± 0,5 |

La nouvelle forme galénique selon l'invention décrite plus haut a fait l'objet d'une étude pharmacocinétique approfondie en comparaison avec une forme à libération immédiate (Isoptine ®) et avec une forme retard actuellement disponible sur le marché (Isoptine Retard ®).

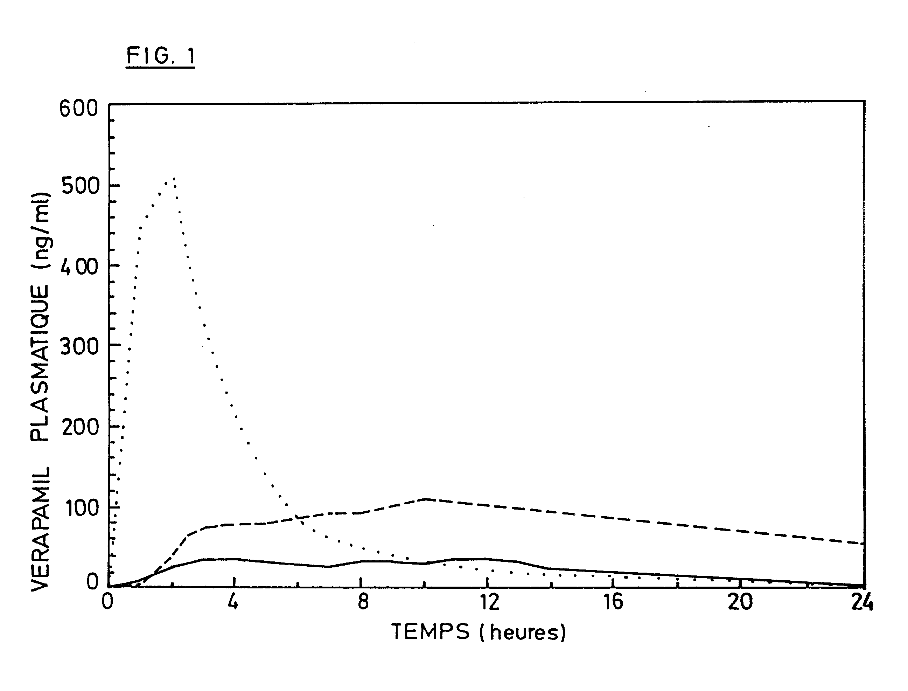

Cinq sujets volontaires sains ont reçu successivement et dans un ordre aléatoire chacune des trois formes à 2 semaines d'intervalle à des doses équivalentes (360 mg de vérapamil). La détermination de la cinétique des concentrations plasmatiques du vérapamil a été effectuée au moyen de 14 à 16 prélèvements et a permis d'obtenir les courbes de la figure 1 ci-annexée.

Sur cette figure 1, la courbe en pointillé est relative à l'Isoptine normale, la courbe en traits interrompus à la forme galénique selon l'exemple 1 et la courbe en trait plein à l'Isoptine® Retard.

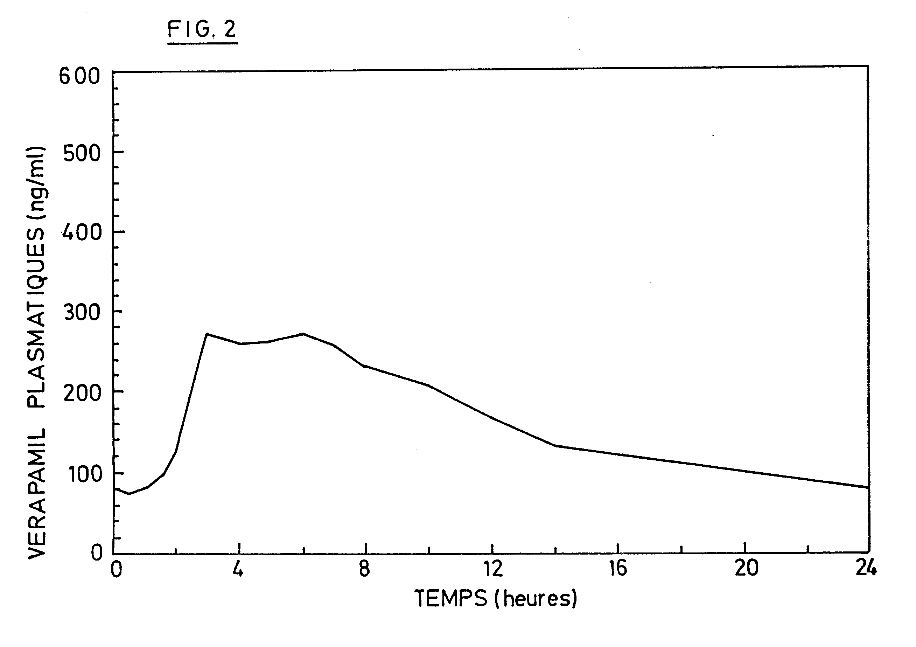

Cinq sujets volontaires sains ont également reçu la nouvelle forme galénique du vérapamil de l'exemple 1 pendant 1 semaine à raison de 1 dose de 360 mg de chlorhydrate de vérapamil toutes les 24 heures. La courbe d'évaluation des taux plasmatiques (exprimés en ng/ml) a été obtenue au moyen de 17 prélèvements sanguins (voir la figure 2).

Au terme de cette étude, les conclusions sont les suivantes :

### 1. Administration en doses uniques (Fig. 1)

### 1.1 Biodisponibilité

D'après les valeurs des surfaces sous les courbes concentrations plasmatiques en ordonnée - temps de 0 à 24 h en abscisse (valeurs non extrapolées à l'infini), la biodisponibilité de la forme galénique de l'exemple 1 s'avère équivalente (88,5 %) à celle du produit à libération immédiate, tandis que celle du produit Isoptine® Retard est nettement inférieure, puisqu'elle ne vaut que 26 % du produit à libération rapide.

### 1.2 Allure des courbes plasmatiques

Les deux formes à libération prolongée fournissent des courbes pratiquement parallèles et dont l'allure est caractéristique de ce type de préparation galénique.

### 2. Administration en doses répétées (Fig. 2)

La courbe moyenne des taux plasmatiques exprimés en ng/ml obtenue après la huitième dose orale quotidienne de 360 mg de chlorhydrate de vérapamil sous la forme galénique de l'exemple 1 se situe, pratiquement pendant 24 heures (c'est-à-dire dans l'intervalle compris entre prises successives), dans une zone thérapeutique optimale.

### EXEMPLE 2

### a. Fabrication des microgranules

On a utilisé les quantités suivantes de produits exprimées en grammes :

| | |
|---|---|
| vérapamil HCl | 480 |
| sucroester WE 15 ® | 60 |
| Avicel ® | 60 |
| eau pour granulation | 165 |

Le mode de préparation était identique à celui de l'exemple 1, si ce n'est que l'on a utilisé l'extrudeuse XTRUDER® (Fuji-Paudal). On a obtenu 524 g de microgranules (rendement : 87,33 %).

### b. Enrobage des microgranules

On a opéré de la même manière que dans l'exemple 1. Sur 500 g de microgranules, on a projeté 305 g de la dispersion aqueuse suivante (quantités exprimées en g) :

| | |
|---|---|
| talc | 50,00 |
| polyvinylpyrrolidone | 7,50 |
| Tween 20 ® | 0,20 |
| eau distillée | 302,30 |
| Eudragit E30D ® | 452,50 |
| Eudragit L30D ® | 187,50 |
| | 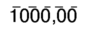 |
| Durée de pistolage : 50 minutes Séchage en étuve à 50°C pendant 24 heures. | |

### c. Forme pharmaceutique

La forme pharmaceutique a été obtenue en procédant comme dans l'exemple 1.

### d. Mesure de la libération du vérapamil des microgranules

En utilisant la méthode et l'appareillage d'analyse décrits dans l'exemple 1, on a obtenu les résultats suivants :

| Temps | % vérapamil HCl dissous ± SD |
|---|---|
| Après 1 h | 1,5 + 0,1 |
| Après 4 h | 29,8 ± 1,0 |
| Après 8 h | 74,2 ± 0,8 |

La nouvelle forme galénique de l'exemple 2 selon l'invention a fait l'objet d'une étude pharmacocinétique approfondie en comparaison avec la forme à libération immédiate (Isoptine ® ) et avec la forme retard actuellement disponible sur le marché (Isoptine Retard ® ).

Cinq sujets volontaires ont reçu successivement chacune des trois formes à 2 semaines d'intervalle à des doses équivalentes (360 mg de vérapamil) et dans un ordre aléatoire.

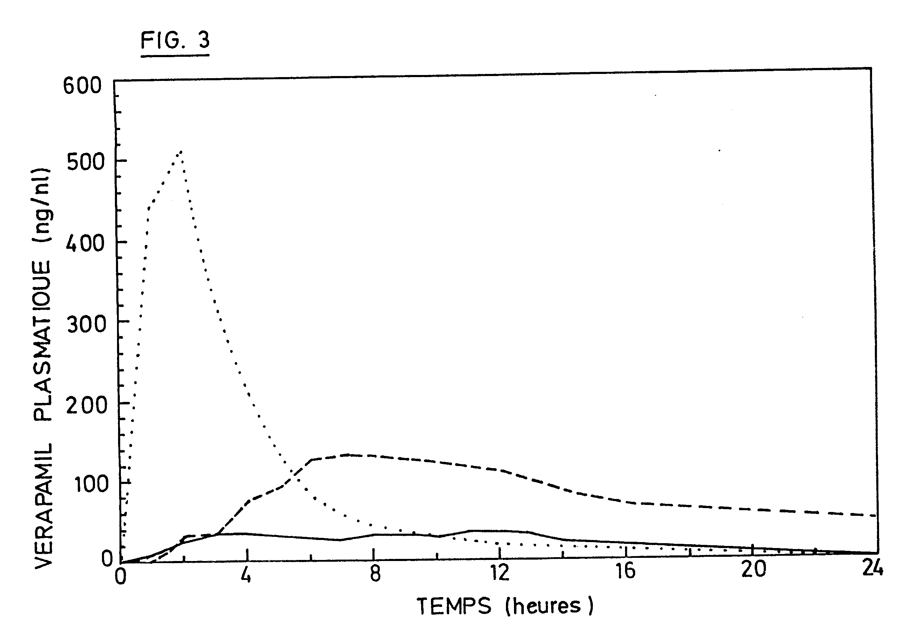

Les taux plasmatiques (exprimés en ng/ml) pour la mesure de l'évolution des concentrations sanguines de vérapamil dans le sang ont été déterminés au moyen de 14 à 16 prélèvements sanguins (voir la figure 3 ci-annexée).

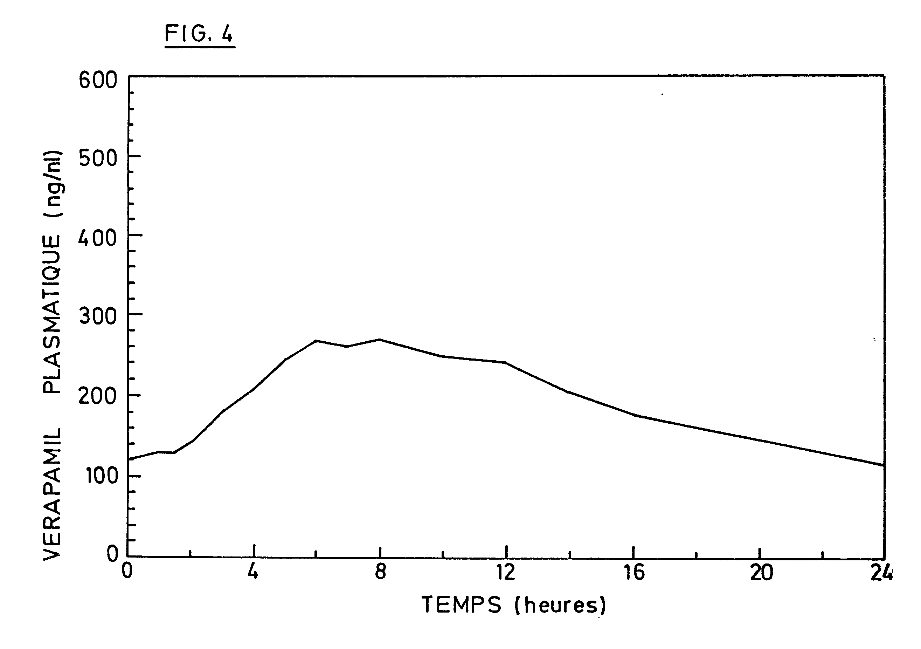

L'évolution des taux plasmatiques de vérapamil a été également mesurée après la huitième dose orale répétée chez 5 sujets sains à raison d'une dose par 24 h de 360 mg calculée en chlorhydrate de vérapamil sous la forme galénique de l'exemple 2 (figure 4).

Au terme de cette étude, on peut donc conclure que :
Aux points de vue des biodisponibilités comparées et des allures des courbes plasmatiques, les conclusions données dans l'étude de l'exemple 1 sont valables pour cet exemple 2, tant dans le cas des doses uniques que dans celui des doses répétées.

### EXEMPLE 3

L'étude de l'influence dans la composition des microgranules d'un agent mouillant a pu être menée, après avoir préparé une forme galénique en utilisant la méthode et l'appareillage décrits dans l'exemple 2, mais en remplaçant l'agent mouillant de l'exemple 2, à savoir le sucroester WE 15, par du lactose qui est une poudre inerte ne possédant pas de propriétés mouillantes, la membrane microporeuse étant quantitativement et qualitativement identique à celle de l'exemple 2.

La forme galénique ainsi obtenue a fait l'objet d'une étude pharmacocinétique approfondie en comparaison avec la forme de l'exemple 2.

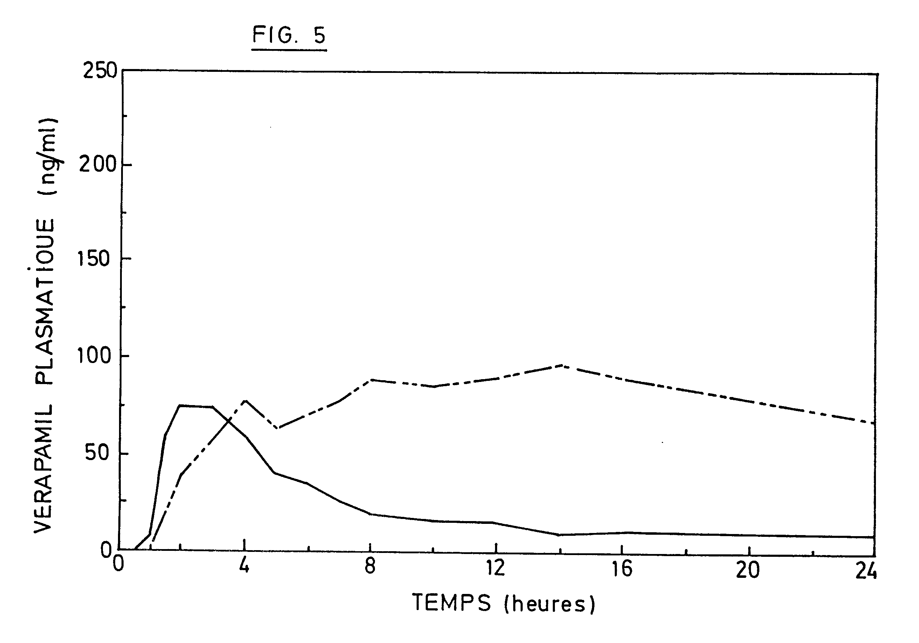

A cette fin, 5 volontaires sains ont reçu successivement chacune des deux formes à 2 semaines d'intervalle à des doses équivalentes (360 mg de chlorhydrate de vérapamil) et dans un ordre aléatoire. Les taux plasmatiques (exprimés en ng/ml) pour la mesure de l'évolution des concentrations sanguines de vérapamil dans le sang ont été déterminés au moyen de 14 prélèvements sanguins (voir figure 5 ci-annexée).

Au terme de cette étude, on peut donc conclure que :
1. La biodisponibilité de la forme sans agent mouillant ne vaut que 29,6 % de celle de la forme avec agent mouillant de l'exemple 2.
2. Bien que la forme galénique sans agent mouillant possède une absorption plus rapide que la forme de l'exemple 2 pendant les deux premières heures, les taux plasmatiques obtenus avec cette forme sans agent mouillant ne sont pas du tout soutenus, extrêmement faibles et insuffisants.

Sur le plan clinique, les caractéristiques de libération lente et continue du vérapamil au départ des microgranules des nouvelles formes galéniques suivant la présente invention ont permis de découvrir les avantages importants suivants :
- Meilleure adhésion du patient à son traitement du fait de la diminution du nombre de prises quotidiennes du médicament. La nouvelle forme galénique ne nécessite en général qu'une seule administration quotidienne, alors qu'un traitement équivalent au moyen de préparations à libération immédiate comporte quatre prises quotidiennes, soit une prise toutes les 6 heures.
- Augmentation de l'efficacité thérapeutique d'une part grâce au maintien de concentrations plasmatiques efficaces durant tout l'espace de temps séparant deux prises successives du médicament, et d'autre part grâce à la suppression de la succession des pics (concentrations plasmatiques momentanément trop fortes) et de vallées (concentrations trop faibles).
- Risques diminués et facilité accrue dans l'adaptation de la posologie individuelle, ceci grâce à une importante diminution des fluctuations des taux plasmatiques d'un patient à l'autre.

Par conséquent, on peut affirmer que les nouvelles formes galéniques faisant l'objet de la présente invention conduisent à un nouveau médicament utile pour le traitement des troubles du rythme cardiaque, de l'hypertension et de l'angine de poitrine ou de toute autre utilisation médicale du vérapamil en administration orale.

## Revendications

1. Forme galénique du vérapamil à libération prolongée de ce composé, caractérisée en ce qu'elle est constituée de microgranules contenant un sel pharmacologiquement acceptable d'addition du vérapamil avec un acide, tel que le chlorhydrate du vérapamil, comme substance active, associé à au moins un mouillant, choisi parmi le saccharose, la polyvinylpyrrolidone, les esters d'acides gras en C₁₂ à C₂₀ du saccharose et du xylose, les glycérides du saccharose, les esters d'acides gras et de polyoxyéthylène, les éthers de sorbitane, les esters de sorbitane, les esters de sorbitane polyoxyéthylénés, les glycérides-polyglycides, les esters d'alcools-polyglycides et les lécithines, ces microgranules étant enrobés d'une membrane microporeuse constituée d'un copolymérisat neutre ester éthylique d'acide acrylique/ester méthylique d'acide méthacrylique dans le rapport 70:30 et de poids moléculaire supérieur à 800.000 associé à au moins un adjuvant choisi parmi les plastifiants, pigments, charges, agents mouillants, agents lubrifiants et agents anti-mousse.

2. Forme galénique du vérapamil suivant la revendication 1, caractérisée en ce que les microgranules contenant la substance active se présentent sous forme de sphérules, dont le diamètre est compris entre 0,05 mm et 3 mm, de préférence entre 0,1 mm et 2 mm.

3. Forme galénique du vérapamil suivant la revendication 1, caractérisée en ce que, dans la membrane microporeuse enrobant les microgranules, le polymère est associé à au moins un adjuvant choisi parmi les suivants :
- plastifiants, tels que la triacétine, le dibutylphtalate, le dibutylsébaçate, les esters d'acide citrique, les polyéthylèneglycols, les polypropylèneglycols et la polyvinylpyrrolidone ;
- pigments éventuellement colorés, tels que les oxydes de fer et l'oxyde de titane ;
- charges, telles que le lactose et le saccharose ;
- mouillants, tels que des esters partiels d'acides gras (acides laurique, palmitique, stéarique et oléique) et d'anhydrides d'hexitols dérivés de sorbitol contenant éventuellement des chaînes polyoxyéthyléniques, ainsi que les polyéthylèneglycols ;
- lubrifiants, tels que le stéarate de magnésium et le talc ;
- agents anti-mousse, tels que l'huile de silicone, et
- agents antistatiques, tels que l'oxyde d'aluminium coloïdal.

4. Forme galénique du vérapamil suivant la revendication 3, caractérisée en ce que la membrane microporeuse contient, outre le polymère, du talc, et/ou du stéarate de magnésium à titre de lubrifiant, de la polyvinylpyrrolidone à titre de plastifiant, du bioxyde de titane à titre de pigment, de l'oxyde d'aluminium colloïdal comme agent antistatique et de l'huile de silicone comme agent anti-mousse.

5. Forme galénique du vérapamil suivant la revendication 1, caractérisée en ce que les microgranules contiennent, en poids, environ 20 à 90 % de chlorhydrate de vérapamil ainsi que 5 à 30 % de sucroesters, 4 à 50 % de cellulose microcristalline, 0 à 35 % de saccharose, 0 à 10 % d'hydroxypropylméthylcellulose et 0 à 15 % de polyvinylvpyrrylidone, à titre de mouillants et excipients.

6. Forme galénique du vérapamil suivant la revendication 5, caractérisée en ce que les microgranules contiennent, en poids, environ 65 % de chlorhydrate de vérapamil, ainsi que 25 % de saccharose, 5 % de cellulose microcristalline, 4 % d'hydroxypropylméthylcellulose, et 1 % de polyvinylpyrrolidone, à titre de mouillants et excipients.

7. Forme galénique du vérapamil suivant la revendication 5, caractérisée en ce que les microgranules contiennent, en poids, environ 80 % de chlorhydrate de vérapamil, ainsi que 10 % de sucroesters comme mouillant et 10 % de cellulose microcristalline comme excipient.

8. Forme galénique du vérapamil suivant la revendication 2, caractérisée en ce que la membrane microporeuse enrobant les microgranules contient en poids, environ 5 à 95 % d'au moins un copolymérisat neutre ester éthylique d'acide acrylique/ester méthylique d'acide méthacrylique dans le rapport 70:30 et de poids moléculaire supérieur à 800.000, 1 à 25 % de talc ou de stéarate de magnésium, 0 à 4 % d'oxyde d'aluminium colloïdal, 0 à 4 % d'oxyde d'aluminium colloïdal, 0 à 15 % de polyvinylpyrrolidone, 0 à 2 % d'huile de silicone et 0 à 0,5 % d'un mouillant du type esters partiels d'acides gras.

9. Forme galénique du vérapamil suivant la revendication 8, caractérisée en ce que la membrane microporeuse enrobant les microgranules contient, en poids, environ 70 à 80 % d'au moins un copolymérisat neutre ester éthylique d'acide acrylique/ester méthylique d'acide méthacrylique dans le rapport 70:30 et de poids moléculaire supérieur à 800.000, 6 % de stéarate de magnésium, 4 à 6 % de polyvinylpyrrolidone et 0,1 % d'esters partiels d'acides gras.

10. Forme galénique du vérapamil suivant la revendication 8, caractérisée en ce que la membrane microporeuse contient, en poids, environ 60 à 80 % d'au moins un copolymérisat neutre ester éthylique d'acide acrylique/ester méthylique d'acide méthacrylique dans le rapport 70:30 et de poids moléculaire supérieur à 800.000, 10 à 25 % de talc, 1 à 5 % de polyvinylpyrrolidone et 0,05 % d'esters partiels d'acides gras.

11. Forme galénique du vérapamil suivant l'une quelconque des revendications précédentes, caractérisée en ce que la membrane microporeuse elle-même contient également du chlorhydrate de vérapamil.

12. Forme galénique du vérapamil suivant l'une quelconque des revendications précédentes, caractérisée en ce que le poids de la membrane microporeuse est de 2 à 35 %, de préférence de 5 à 22 % de celui des microgranules qu'elle enrobe.

13. Médicament contenant du vérapamil à administrer par voie buccale, caractérisé en ce qu'il contient une forme galénique suivant l'une quelconque des revendications précédentes, dans une gélule, un sachet ou un distributeur de doses.

14. Médicament suivant la revendication 13, caractérisé en ce qu'il se présente sous forme de doses unitaires contenant de 20 à 400 mg, de préférence de 50 à 360 mg de vérapamil.

15. Procédé de fabrication d'une nouvelle forme galénique du vérapamil suivant l'une quelconque des revendications 1 à 12, caractérisé en ce qu'il consiste à préparer au préalable des microgranules et à les enrober d'une membrane microporeuse, les microgranules et les membranes microporeuses présentant les compositions et caractéristiques indiquées dans l'une ou l'autre des revendications 1 à 12.

16. Procédé suivant la revendication 15, caractérisé en ce qu'il consiste à mélanger du chlorhydrate de vérapamil et le ou les mouillants et excipients choisis sous forme finement divisée ou fondue, ou en solution, en présence d'un solvant, tel que de l'eau ou un mélange hydroalcoolique, de manière à obtenir une masse plastique ou pâte extrudable, puis à extruder la masse plastique sous forme de cylindres, à rendre ces cylindres sphériques et à sécher les microgranules obtenus.

17. Procédé suivant la revendication 16, caractérisé en ce qu'on utilise une extrudeuse à filière d'un diamètre compris entre 0,1 et 3 mm, de préférence entre 0,5 et 2 mm.

18. Procédé suivant l'une quelconque des revendications 15 à 17, caractérisé en ce qu'il consiste à pistoler et/ou à saupoudrer des noyaux obtenus par agglomération de chlorhydrate de vérapamil mélangé éventuellement à au moins un excipient, à l'aide d'une solution ou dispersion aqueuse de chlorhydrate de vérapamil et d'au moins un mouillant, dans une turbine connue de dragéification ou un appareil de granulation.

19. Procédé suivant l'une quelconque des revendications 15 à 18, caractérisé en ce qu'on applique la membrane microporeuse sur les microgranules en pulvérisant une dispersion aqueuse ou hydroalcoolique d'au moins un des polymères précités et d'au moins un des adjuvants susdits sur les microgranules.

20. Procédé suivant l'une quelconque des revendications 15 à 19, caractérisé en ce qu'on pulvérise sur les microgranules une dispersion aqueuse contenant en poids :
| | |
|---|---|
| - 10 à 45 % | de copolymérisat neutre ester éthylique d'acide acrylique/ester méthylique d'acide méthacrylique dans le rapport 70:30 et de poids moléculaire supérieur à 800.000 ; |
| - 5 à 70 % | de polymérisat anionique d'acide méthacrylique et d'ester méthylique d'acide méthacrylique ; |
| - 1 à 25 % | de talc (lubrifiant) ; |
| - 1 à 15 % | de polyvinylpyrrolidone (plastifiant) ; |
| - 0,05 à 4 % | d'oxyde d'aluminium colloïdal (agent antimousse), et |
| - 10 à 70 % | d'eau. |

## Claims

1. Galenic form of verapamil with sustained release of said compound, characterized in that it consists of microgranules containing a pharmacologically acceptable verapamil addition salt with an acid, such as verapamil chlorhydrate, as active substance, associated to at least one wetting agent selected from sucrose, polyvinylpyrrolidone, C₁₂ to C₂₀ fatty acid esters of sucrose and xylose, sucrose glycerides, esters of fatty acid and polyoxyethylene, sorbitan ethers, sorbitan esters, polyoxyethylene sorbitan esters, polyglycides-glycerides, poly-glycides-alcohols esters and lecithins, said microgranules being coated with a microporous membrane constituted of neutral acrylic acid ethylester/methacrylic acid methylester copolymerisate in the ratio 70 : 30, and having a molecular weight above 800,000 associated to at least a pharmacologically acceptable adjuvant selected from plastifying agents, pigments, fillers, wetting agents, lubricants and antifoaming agents.

2. Galenic form of verapamil according to claim 1, characterized in that said active substance containing microgranules are present in the form of spherules, the diameter of which is comprised between 0.05 mm and 3 mm, preferably between 0.1 mm and 2 mm.

3. Galenic form of verapamil according to claim 1, characterized in that within the microporous membrane coating said microgranules, the polymer is associated to at least an adjuvant selected from the following :
- plastifying agents, such as triacetine, dibutylphthalate, dibutylsebaçate, citric acid esters, polyethyleneglycols, polypropyleneglycols and polyvinylpyrrolidone ;
- possibly coloured pigments, such as iron oxides and titanium oxide ;
- fillers, such as lactose and sucrose ;
- wetting agents, such as fatty acid partial esters (lauric, palmitic, stearic and oleic acids) and anhydrydes of hexitols derivated from sorbitol containing possibly polyoxyethylenic chains, as well as polyethylene glycols ;
- lubricants, such as magnesium stearate and talc ;
- antifoaming agents, such as silicone oil, and
- antistatic agents, such as colloidal aluminium oxide.

4. Galenic form of verapamil according to claim 3, characterized in that the microporous membrane contains, in addition to the polymer, talc and/or magnesium stearate as a lubricant, polyvinylpyrrolidone as a plastifying agent, titanium bioxide as a pigment, colloidal aluminium oxide as an antistatic agent and silicone as an antifoaming agent.

5. Galenic form of verapamil according to claim 1, characterized in that the microgranules contain, by weight, about 20 to 90% of verapamil chlorhydrate, as well as 5 to 30% of sucroesters, 4 to 50% of microcristalline cellulose, 0 to 35% sucrose, 0 to 10% hydroxypropylmethyl cellulose and 0 to 15% polyvinylpyrrolidone, as wetting agents and excipients.

6. Galenic form of verapamil according to claim 5, characterized in that the microgranules contain, by weight, about 65% verapamil chlorhydrate, as well as 25% sucrose, 5% of microcristalline cellulose, 4% hydroxypropylmethyl cellulose and 1% polyvinylpyrrolidone, as wetting agents and excipients.

7. Galenic form of verapamil according to claim 5, characterized in that the microgranules contain, by weight, about 80% verapamil chlorhydrate, as well as 10% sucroesters as wetting agent and 10% of microcristalline cellulose as excipient.

8. Galenic form of verapamil according to claim 2, characterized in that the microporous membrane coating said microgranules contains, by weight, about 5 to 95% of at least a neutral acrylic acid ethylester/methacrylic acid methylester copolymerisate in the ratio 70 : 30, and having a molecular weight above 800,000, 1 to 25% talc or magnesium stearate, 0 to 4% colloidal aluminium oxide, 0 to 15% polyvinylpyrrolidone, 0 to 2% silicone oil and 0 to 0.5% of a wetting agent of the type of partial esters of fatty acids.

9. Galenic form of verapamil according to claim 8, characterized in that the microporous membrane coating said microgranules comprises, by weight, about 70 to 80% of at least a neutral acrylic acid ethylester/methacrylic acid methylester copolymerisate in the ratio 70 : 30, and having a molecular weight above 800,000, 6% magnesium stearate, 4 to 6% titanium bioxide, 4 to 6 polyvinylpyrrolidone and 0,1% partial esters of fatty acids.

10. Galenic form of verapamil according to claim 8, characterized in that the microporous membrane contains by weight, about 60 to 80% of at least a neutral acrylic acid ethylester/methacrylic acid methylester copolymerisate in the ratio 70 : 30, and having a molecular weight above 800,000, 10 to 25% talc, 1 to 5% polyvinylpyrrolidone and 0.05% partial esters of fatty acids.

11. Galenic form of verapamil according to anyone of the preceeding claims, characterized in that the microporous membrane itself contains also verapamil chlorhydrate.

12. Galenic form of verapamil according to anyone of the preceding claims, characterized in that the microporous membrane weight makes from 2 to 35%, preferably 5 to 22%, of the weight of the microgranules coated by same.

13. Medicine containing verapamil to be administrated through oral way, characterized in that it comprises a galenic form according to anyone of the preceding claims, into a capsule, a bag or a dosage dispenser.

14. Medicine according to claim 13, characterized in that said medicine is in the form of unit dosages containing from 20 to 400 mg, preferably 50 to 360 mg, verapamil.

15. Process for manufacturing a novel galenic form of verapamil according to anyone of the claims 1 to 12, characterized in that it consists of previously preparing microgranules and coating same with a microporous membrane, the microgranules and the microporous membranes having the composition and features indicated in one or another of the claims 1 to 12.

16. Process according to claim 15, characterized in that it consists of mixing verapamil chlorhydrate and wetting agent(s) and carrier(s) or excipient(s) selected in melted or finely divided form, or in solution, in the presence of a solvent, such as water or an hydroalcoholic mixture, so as to obtain an extrudable paste or plastic mass, thereafter extruding said plastic mass in the form of cylinders, rendering said cylinders spherical and drying the obtained microgranules.

17. Process according to claim 16, characterized in that an extruder with a die having a diameter comprised between 0.1 and 3 mm, preferably between 0.5 and 2 mm is used.

18. Process according to anyone of the claims 15 to 17, characterized in that it consists in gunspraying and/or pulverizing cores obtained through agglomeration of verapamil chlorhydrate possibly mixed to at least one excipient, with an aqueous dispersion or solution of verapamil chlorhydrate and at least one wetting agent, in a known pilling turbin or a granulation apparatus.

19. Process according to anyone of the claims 15 to 18, characterized in that said microporous membrane onto said microgranules is applied by pulverizing an hydroalcoholic or aqueous dispersion of at least one of the abovementioned polymers and of at least one of the abovementioned adjuvants onto the microgranules.

20. Process according to anyone of the claims 15 to 19, characterized in that onto the microgranules an aqueous dispersion containing, by weight :
| | |
|---|---|
| - 10 to 45% | of neutral acrylic acid ethylester/ methacrylic acid methylester copolymerisate in the ratio 70 : 30, and having a molecular weight above 800,000 ; |
| - 5 to 70% | of anionic methacrylic acid and methacrylic acid methylester polymerisate ; |
| - 1 to 25% | talc (lubricant) ; |
| - 1 to 15% | polyvinylpyrrolidone (plastifying agent) ; |
| - 0.05 to 4% | colloidal aluminium oxide (antifoaming agent) and |
| - 10 to 70% | water, |
is pulverized

## Patentansprüche

1. Galenische Form des Verapamil zur Langzeitfreisetzung dieser Verbindung, dadurch gekennzeichnet, daß sie aus Mikrogranula besteht, die ein pharmakologisch annehmbares Salz aus der Addition des Verapamil mit einer Säure enthalten, wie das Verapamil-hydrochlorid, als aktive Substanz, die wenigstens an ein Netzmittel gebunden ist, das aus Saccharose, Polyvinylpyrrolidon, den Fettsäureestern von C₁₂ bis C₂₀ der Saccharose und der Xylose, den Glyceriden der Saccharose, den Estern von Fettsäuren und Polyoxyethylen, den Ethern von Sorbitan, den Estern von Sorbitan, den Estern von Polyoxyethylensorbitan, den Glycerinpolyglyciden, den Estern von Polyglycidalkoholen und den Lecithinen auszuwählen ist, wobei diese Mikrogranula von einer mikroporösen Membran umhüllt sind, die aus einem neutralen Copolymerisat Acrylsäureethylester/Methacrylsäuremethylester im Verhältnis 70:30 und einem Molgewicht über 800 000 besteht, in Verbindung mit wenigstens einem Adjuvans aus dem Bereich der Weichmacher, Pigmente, Füllstoffe, Netzmittel, Gleitmittel und Antischaummittel.

2. Galenische Form des Verapamil gemäß Anspruch 1, dadurch gekennzeichnet, daß die die aktive Substanz enthaltenden Mikrogranula in Kügelchen vorliegen, deren Durchmesser zwischen 0,05 mm und 3 mm liegt, vorzugsweise zwischen 0,1 mm und 2 mm.

3. Galenische Form des Verapamil gemäß Anspruch 1, dadurch gekennzeichnet, daß das Polymere in der mikroporösen Membran, die die Mikrogranula umgibt, an wenigstens ein Adjuvans aus den folgenden assoziiert ist:
- Weichmacher, wie Glycerintriacetat, Dibutylphthalat, Dibutylsebacat, Citronensäureester, Polyethylenglycole, Polypropylenglycole und Polyvinylpyrrolidon;
- Pigmente, gegebenenfalls farbig, wie Eisen- oder Titanoxide;
- Füllstoffe wie Lactose und Saccharose;
- Netzmittel, wie partiell veresterte Fettsäuren (Laurinsäure, Palmitinsäure, Stearin - und Ölsäure) und Anhydride von Hexiten, die sich von Sorbit ableiten und die gegebenfalls Polyoxyethylenketten enthalten, sowie auch die Polyethylenglycole;
- Gleitmittel, wie Magnesiumstearat und Talkum;
- Antischaummittel, wie Siliconöl und
- antistatische Mittel, wie kolloidales Aluminiumoxid.

4. Galenische Form des Verapamil nach Anspruch 3, dadurch gekennzeichnet, daß die mikroporöse Membran außer dem Polymeren, Talkum und/oder Magnesiumstearat als Gleitmittel, Polyvinylpyrrolidon als Weichmacher, Titandioxid als Pigment, kolloidales Aluminumoxid als antistatisches Mittel und Siliconöl als Antischaummittel enthält.

5. Galenische Form des Verapamil nach Anspruch 1, dadurch gekennzeichnet, daß die Mikrogranula bezogen auf ihr Gewicht ungefähr 20 bis 90 % Verapamil-hydrochlorid enthalten, sowie 5 bis 30 % Zuckerester, 4 bis 50 % mikrokristalline Cellulose, 0 bis 35 % Saccharose, 0 bis 10 % Hydroxypropylmethylcellulose und 0 bis 15% Polyvinylpyrrolidon als Netzmittel und Hilfsstoffe.

6. Galenische Form des Verapamil nach Anspruch 5, dadurch gekennzeichnet, daß die Mikrogranula bezogen auf ihr Gewicht ungefähr 65 % Verapamil-hydrochlorid enthalten, sowie 25 % Saccharose, 5 % mikrokristalline Cellulose, 4 % Hydroxypropylmethylcellulose und 1 % Polyvinylpyrrolidon als Netzmittel und Hilfsstoffe.

7. Galenische Form des Verapamil nach Anspruch 5, dadurch gekennzeichnet, daß die Mikrogranula bezogen auf ihr Gewicht ungefähr 80 % Verapamil-hydrochlorid enthalten, sowie 10 % Zuckerester als Netzmittel und 10 % mikrokristalline Cellulose als Hilfsstoff.

8. Galenische Form des Verapamil nach Anspruch 2, dadurch gekennzeichnet, daß die mikroporöse Membran, die die Mikrogranula umhüllt, bezogen auf ihr Gewicht ungefähr 5 bis 95 % von wenigstens einem neutralen Copolymerisat aus Acrylsäureethylester/ Methacrylsäuremethylester im Verhältnis 70:30 enthält, mit einem Molekulargewicht über 800 000, 1 bis 25 % Talkum oder Magnesiumstearat, 0 bis 4 % kolloidales Aluminiumoxid, 0 bis 15 % Polyvinylpyrrolidon, 0 bis 2 % Siliconöl und 0 bis 0,5 % eines Netzmittels des Typus partiell veresterter Fettsäuren.

9. Galenische Form des Verapamil nach Anspruch 8, dadurch gekennzeichnet, daß die mikroporöse Membran, die die Mikrogranula umhüllt, bezogen auf ihr Gewicht ungefähr 70 bis 80 % wenigstens eines neutralen Copolymerisates aus Acrylsäureethylester/ Methacrylsäuremethylester im Verhältnis 70:30 enthält, mit einem Molekulargewicht über 800 000, 6 % Magnesiumstearat, 4 bis 6 % Polyvinylpyrrolidon und 0,1 % partiell veresterter Fettsäuren.

10. Galenische Form des Verapamil nach Anspruch 8, dadurch gekennzeichnet, daß die mikroporöse Membran, die die Mikrogranula umhüllt, bezogen auf ihr Gewicht ungefähr 60 bis 80 % von wenigstens einem neutralen Copolymerisat aus Acrylsäureethylester/ Methacrylsäuremethylester im Verhältnis 70:30 enthält mit einem Molekulargewicht über 800 000, 10 bis 25 % Talkum, 1 bis 5 % Polyvinylpyrrolidon und 0,05 % partiell veresterte Fettsäuren.

11. Galenische Form des Verapamil nach irgendeinem der vorstehenden Ansprüche, dadurch gekennzeichnet, daß die mikroporöse Membran selbst ebenfalls Verapamilhydrochlorid enthält.

12. Galenische Form des Verapamil nach irgendeinem der vorstehenden Ansprüche, dadurch gekennzeichnet, daß die Masse der mikroporösen Membran 2 bis 35 %, vorzugsweise 5 bis 22 % der Mikrogranula ausmacht, die sie umgibt.

13. Medikament, das Verapamil enthält und per os verabreicht wird, dadurch gekennzeichnet, daß es eine galenische Form enthält, gemäß irgendeinem der vorhergehenden Ansprüche, in einer Gelatinekapsel, einem Beutel oder einer Dosiervorrichtung.

14. Medikament gemäß Anspruch 13, dadurch gekennzeichnet, daß es in Einzeldosen 20 bis 400 mg, vorzugsweise 50 bis 360 mg Verapamil enthält.

15. Verfahren zur Herstellung einer neuen galenischen Form des Verapamil gemäß irgendeinem der Ansprüche 1 bis 12, dadurch gekennzeichnet, daß es in der vorausgehenden Herstellung der Mikrogranula besteht und deren Ummantelung mit einer mikroporösen Membran, wobei die Mikrogranula und die mikroporösen Membranen die Bestandteile und die Charakteristika enthalten, die in irgendeinem der Ansprüche 1 bis 12 beschrieben sind.

16. Verfahren gemäß Anspruch 15, dadurch gekennzeichnet, daß es Verapamil-hydrochlorid mit dem oder den Netzmitteln und Hilfsstoffen mischt, die gewählt wurden in fein verteilter oder geschmolzener Form, oder in Lösung bei Anwesenheit eines Lösungsmittels, wie Wasser oder ein Wasser- Alkoholgemisch, so daß eine plastische Masse oder eine preßbare Paste erhalten wird, die weiche Masse in Zylinder gepreßt, diese Zylinder zu Kugeln verarbeitet und die erhaltenen Mikrogranula getrocknet werden.

17. Verfahren gemäß Anspruch 16, dadurch gekennzeichnet, daß man eine Extruderdüse mit einem Durchmesser zwischen 0,1 und 3 mm, vorzugsweise zwischen 0,5 und 2 mm benutzt.

18. Verfahren gemäß irgendeinem der Ansprüche 15 bis 17, dadurch gekennzeichnet, daß es im Besprühen und/oder Anpudern von Kernen besteht, die durch die Agglomeration von Verapamil-hydrochlorid, gegebenenfalls vermischt mit wenigstens einem Hilfsstoff, mit Hilfe einer Lösung oder wässrigen Dispersion von Verapamil-hydrochlorid und von wenigstens einem Netzmittel in einer bekannten Dragiermaschine oder einem Granulator erhalten wurden.

19. Verfahren gemäß irgendeinem der Ansprüche 15 bis 18, dadurch gekennzeichnet, daß man die mikroporöse Membran auf die Mikrogranula aufbringt, indem man eine wässrige oder wasserhaltige alkoholische Dispersion von wenigstens einem der aufgeführten Polymeren und von wenigstens einem der oben genannten Adjuvantien auf den Mikrogranula zerstäubt.

20. Verfahren gemäß irgendeinem der Ansprüche 15 bis 19, dadurch gekennzeichnet, daß auf die Mikrogranula eine wässrige Dispersion mit folgenden Gewichtsanteilen zerstäubt wird:
- 10 bis 45 % neutrales Copolymerisat aus Acrylsäureethylester/Methacrylsäuremethylester im Verhältnis 70:30 und Molgewicht über 800 000;
- 5 bis 70 % anionisches Polymerisat aus Methacrylsäure und Methacrylsäuremethylester;
- 1 bis 25 % Talkum (Gleitmittel);
- 1 bis 15 % Polyvinylpyrrolidon (Weichmacher);
- 0,05 bis 4 % kolloidales Aluminiumoxid (Antischaummittel), und
- 10 bis 70 % Wasser
